# EUROPEAN PATENT APPLICATION

(11) **EP 4 759 280 A1**
(43) Date of publication of application: **17.06.2026**
(21) Application number: 24879764.9
(22) Date of filing: 17.10.2024
(51) Int. Cl.: A61F 9/007

(54) **ANNULAR DEVICE**

(30) Priority: 20.10.2023 JP 2023180706
(71) Applicant: National University Corporation Hokkaido University, Hokkaido 060-0808 (JP); Seed Co., Ltd., Tokyo 113-8402 (JP)
(72) Inventor: SATO, Takao, Tokyo 113-8402 (JP); YAMAZAKI, Yoshiko, Tokyo 113-8402 (JP); MATSUNAGA, Toru, Tokyo 113-8402 (JP); ISHIDA, Susumu, Sapporo-shi, Hokkaido 060-0808 (JP); OHGUCHI, Takeshi, Sapporo-shi, Hokkaido 060-0808 (JP); TAGAWA, Yoshiaki, Sapporo-shi, Hokkaido 060-0808 (JP)
(74) Representative: Schulz Junghans Patentanwälte PartGmbB
(86) International application number: PCT/JP2024/036934
(87) International publication number: WO 2025/084343

(57) **Abstract**

The objective of the present invention is to provide an annular device that can suppress the effect of frictional forces generated between the annular device and the eyelid conjunctiva during blinking and that can exhibit excellent stability on the eye when worn, as compared to the previous annular devices. The objective can be achieved by an annular device configured to be worn over a surface of a sclera, comprising: an opening configured to expose a cornea; and a body portion having a slab-off region and a non-slab-off region, wherein the slab-off region is formed such that a maximum thickness thereof gradually decreases from a right end side toward upper and lower end sides and from a left end side toward upper and lower end sides; and the like.

## Description

### Technical Field

The invention relates to an annular device configured to be worn over a surface of a sclera.

### Background Art

As a medication method for treating an eye disease, there has been developed a method using a drug delivery system-type contact lens (DDSCL). In such a method, the DDSCL contains a drug and, when being worn, provides controlled release of the drug to an ocular tissue. Since the DDSCL can cover the surface of a sclera, this method has been understood to be able to deliver a drug at an effective concentration to a tissue in the anterior eye segment such as the cornea and the aqueous humor and to a tissue in the exterior eye segment such as the conjunctiva and tear.

Such a method using a DDSCL, however, is difficult to deliver a drug selectively to a tissue in the posterior eye segment such as the retina, the choroid, the sclera and the vitreous, which are located posterior to the crystalline lens. The present inventors have developed an annular (ring-shaped) device with a body portion covering a surface of a sclera and an opening in the center configured to expose the cornea, which can deliver a drug efficiently to a tissue in the posterior eye segment (see Patent Document 1).

The annular device described in Patent Document 1 is configured to have a maximum thickness in an intermediate portion formed between inner and outer rim portions located at both ends of the width of the body portion in order to obtain good wearing sensation and stability on the eyeball when worn in the eye. However, the annular device described in Patent Document 1 has been insufficient in stability on the eyeball.

Therefore, the present inventors have developed an annular device configured to further add at least one approximately encircling groove on a back surface of the intermediate portion to the annual device described in Patent Document 1 (see Patent Document 2). The annular device described in Patent Document 2 had the groove on the back surface of the intermediate portion so as to suppress its mobility on the surface of the sclera, resulting in improved stability on the eyeball.

On the other hand, a slab-off process has been employed in eyeglass lenses and contact lenses. The slab-off process refers to a process in which a portion of a lens is removed in thickness from a peripheral portion toward a center portion. The contact lens produced by the slab-off process can be stably positioned on the eye with a slab-off region having a reduced thickness in a vertical direction of the lens (see Patent Document 3).

### Citation List

### Patent Documents

[Patent document 1] WO 2010/092735 A
[Patent document 2] WO 2014/192853 A
[Patent Document 3] JP 2009-169104 A

### Summary of the Invention

### Problems to be Solved by the Invention

The annular device described in Patent Document 2 is more stable on the eyeball as compared to the annular device described in Patent Document 1. However, even when using the annular device described in Patent Document 2, there remain problems in achieving further stability on the eyeball, since the annular device may become misaligned due to frictional forces generated between the eyelid conjunctiva and the annular device during blinking. If the annular device is misaligned on the eye while being worn, the wearer may experience a foreign body sensation, as well as a risk of inducing ocular disorders such as scarring on the corneal surface.

The contact lens described in Patent Document 3 has a slab-off region, thereby enabling stable positioning on the ocular surface. However, a contact lens worn on the corneal surface differs from an annular device worn on the scleral surface with respect to frictional forces generated during blinking and their effects. Furthermore, Patent Document 3 fails to provide any specific description of a slab-off region structure.

Therefore, an objective of the present invention is to provide an annular device that can suppress the effects of frictional forces generated between the annular device and the eyelid conjunctiva during blinking and that can exhibit excellent stability on the eyeball when worn, as compared to the annular device described in Patent Document 2.

### Means of Solving the Problems

The present inventors have conducted intensive research on the shape of an annular device to reduce the effects caused by blinking during wearing, and have found that an annular device including a slab-off region formed such that a maximum thickness of a portion that contacts the eyelid conjunctiva when worn gradually decreases from one side toward the other can reduce frictional forces caused by rubbing between the eyelid conjunctiva and the annular device surface, thereby preventing misalignment caused by blinking. Furthermore, the present inventors have surprisingly found that by increasing a maximum thickness of a non-slab-off region, which is different from the slab-off region, in a body portion of the annular device as compared to the annular devices described in Patent Documents 1 and 2, and by defining a predetermined relationship between maximum thicknesses of the slab-off region and the non-slab-off region, the annular device can reduce frictional forces during blinking, can effectively suppress misalignment of the annular device, and further can improve handling and wearing sensations. Based on these findings, the present inventors have succeeded in inventing an annular device that can solve the problems of the present invention. As such, the present invention has been completed on the basis of the findings and successful examples.

According to the present invention, there are provided annular devices in the following aspects:
[1] An annular device configured to be worn over a surface of a sclera, comprising:
   an opening configured to expose a cornea; and
   a body portion having a slab-off region and a non-slab-off region,
   wherein the slab-off region is formed such that a maximum thickness thereof gradually decreases from a lower end side toward an upper end side.
[2] The annular device according to the item [1], wherein the slab-off region is line-symmetrical with respect to a vertical line as an axis of symmetry.
[3] The annular device according to the item [2], wherein the slab-off region is formed such that a maximum thickness thereof gradually decreases from a lower end side toward an upper end side, starting from a position where a center angle is in a range of 40° to 170°.
[4] An annular device configured to be worn over a surface of a sclera, comprising:
   an opening configured to expose a cornea; and
   a body portion having a slab-off region and a non-slab-off region,
   wherein the slab-off region is formed such that a maximum thickness thereof gradually decreases from a right end side toward an upper end side and a lower end side, and from a left end side toward an upper end side and a lower end side.
[5] The annular device according to the item [4], wherein the slab-off region is line-symmetrical with respect to a vertical line as an axis of symmetry.
[6] The annular device according to the item [5], wherein the slab-off region is formed such that a maximum thickness thereof gradually decreases from a right end side toward an upper end side, starting from a position where a center angle is in a range of 40° to 85°, and from a right end side toward a lower end side, starting from a position where a center angle is in a range of 95° to 140°.
[7] The annular device according to any one of the items [1] to [6], wherein the slab-off region has a surface area of 25% to 70% relative to a total surface area of the body portion.
[8] The annular device according to any one of the items [1] to [7], wherein a maximum thickness of the non-slab-off region of the body portion is 120% to 450% relative to a minimum maximum thickness of the slab-off region.
[9] The annular device according to any one of the items [1] to [8], wherein the body portion has one or more approximately encircling grooves on a back surface thereof.

### Effects of the Invention

The annular device according to one embodiment of the present invention has a slab-off region in a specific area of the annular device located on an upper eyelid side, or on upper and lower eyelid sides, thereby suppressing the effects of frictional forces generated between the annular device and the eyelid conjunctiva during blinking, and exhibiting excellent stability on the eyeball when worn. As a result, it is possible to suppress misalignment of the annular device, thereby reducing the occurrence of foreign body sensation and ocular disorders when the annular device is worn. Furthermore, according to the annular device according to one embodiment of the present invention, by enlarging a maximum thickness of a non-slab-off region in a body portion of the annular device while defining a predetermined relationship between maximum thicknesses of the slab-off region and the non-slab-off region of the annular device, it is possible to reduce frictional forces during blinking, effectively suppress misalignment of the annular device, and further improve handling and wearing sensations of the annular device. Furthermore, when used as a drug-releasing annular device, the annular device according to one embodiment of the present invention can deliver the drug to a desired portion on the eye.

### Brief Description of the Drawing

[Figure 1A] Figure 1A shows a plan view and cross-sectional views of the annular device, according to one embodiment of the first form.
[Figure 1B] Figure 1B shows a plan view and cross-sectional views, which show a cross-section for each center angle, of the annular device, according to one embodiment of the first from.
[Figure 2A] Figure 2A shows a plan view and cross-sectional views of the annular device, according to one embodiment of the second form.
[Figure 2B] Figure 2B shows a plan view and cross-sectional views of the annular device having grooves on the back surface, according to one embodiment of the second form.
[Figure 3] Figure 3 shows photographs showing the annular device of Example 13 as described below.
[Figure 4] Figure 4 shows photographs showing the eye area taken from the front when wearing the annular device of Example 1 as described below.
[Figure 5] Figure 5 shows cross-sectional images obtained by cutting the annular device of Example 13 along a vertical line and a horizontal line, respectively, and photographed by optical coherence tomography (OCT).

### [Description of Embodiments]

While each aspect that forms one embodiment of the present invention will now be described in detail, the present invention is not limited only by the matters of this section, and may take various forms to the extent that its objective can be achieved.

Unless otherwise specified, each term used herein is used in the meaning commonly used by those skilled in the art, in particular in the technical fields relating to ocular lens such as contact lens and annular lens, and should not be construed to have any meaning that is unduly limiting. Also, any speculations and theories herein are made on the basis of the knowledge and experiences of the present inventors and as such, the present invention is not bound by any such speculations and theories.

The term "and/or" as used herein means either any one of, any combination of two or more of, or combination of all of listed related items.

### [Aspects of annular device]

According to an aspect of the invention, there is provided an annular device configured to be worn over a surface of a sclera, including an opening configured to exposes a cornea, and a body portion having a slab-off region, and a non-slab-off region that is a region other than the slab-off region. Depending on the location of the slab-off region, the annular devices are broadly classified into first and second forms.

As used herein, when the annular device is worn, the direction of the upper eyelid side is referred to as an upper end side, while the direction of the lower eyelid side as a lower end side. In the figures as described below, the upper direction of the annular device is referred to as an upper end side, the right direction as a right end side, the lower direction as a lower end side, and the left direction as a left end side. Furthermore, the visible side in the figures represents a front surface of the annular device, while the side visible from the back of the figures represents a back surface of the annular device. The annular device is worn to a scleral surface from the back side. When worn, the back surface of the annular device adheres to the scleral surface of the ocular tissue, i.e., the surface of the bulbar conjunctiva that covers the sclera.

The uppermost location on the upper end side of the annular device is referred to as an upper end, the rightmost location on the right end side as a right end, the lowest location on the lower end side as a lower end, and the leftmost location on the left end side as a left end. The point located at the center of the annular device, i.e., the point at the center of the opening, is referred to as a center point. The angle from the position of a point toward the position of the upper end clockwise around the center point is referred to as a center angle. The center angle of the upper end position is 0°, the center angle of the right end position is 90°, the center angle of the lower end position is 180°, and the center angle of the left end position is 270°.

The line passing through the upper and lower ends of the annular device represents a vertical line, while the line passing through the right and left ends of the annular device represents a horizontal line. The vertical and horizontal lines intersect at the center point.

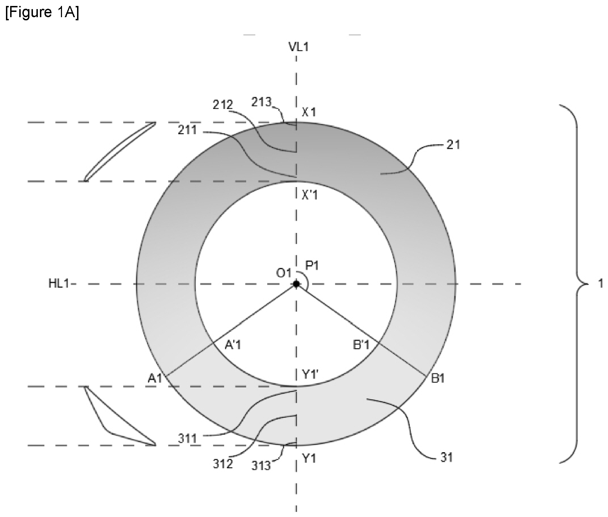

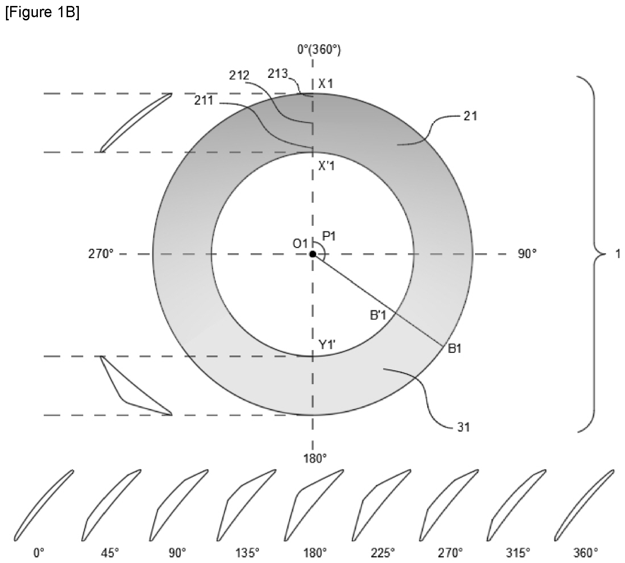

The first form of annular device is characterized in that the annular device is configured to have a slab-off region that is located on the upper eyelid side when worn. The slab-off region on the upper end side of the annular device may reduce frictional forces caused by rubbing between the eyelid conjunctiva of the upper eyelid side and the front surface of the annular device when worn, thereby suppressing misalignment of the annular device caused by blinking. Figures 1A and 1B represent plan views schematically illustrating one embodiment of the first form of annular device.

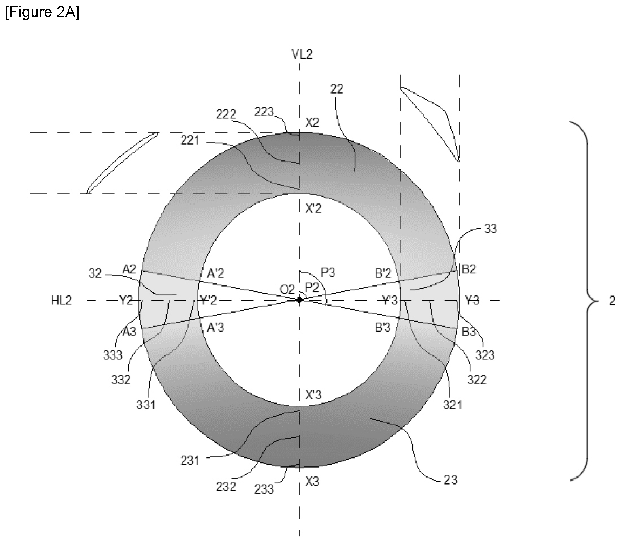

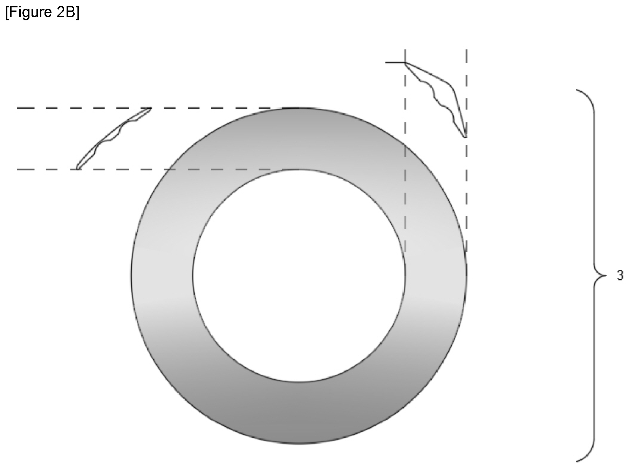

The second form of annular device is characterized in that the annular device is configured to have a first slab-off region located on the upper eyelid side, and a second slab-off region located on the lower eyelid side, when worn. The two slab-off regions, which are located on the upper and lower eyelid sides when worn, may firmly restrain rotational movement of the annular device, thereby favorably improving suppression of misalignment of the annular device during blinking. Figures 2A and 2B represent plan views schematically illustrating one embodiment of the second form of annular device.

The details of each aspect of the present invention are described below with reference to the figures. However, the present invention is not limited to the embodiments illustrated in the figures, as long as the objective of the present invention can be achieved.

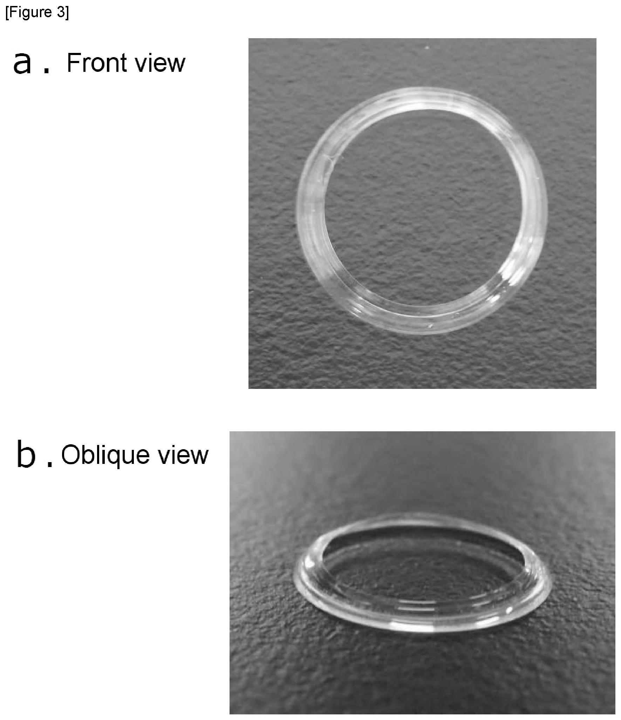

Figure 3 represents photographs showing the annular device of Example 13 as described below. As shown by the front and oblique views, the structure of an annular device to be worn on the surface of the sclera is substantially different from that of a conventional contact lens to be worn on the cornea, although both are classified as ocular lenses. Therefore, the annular device has a circular (doughnut-shaped) configuration with an opening configured to expose the cornea, and has a larger outer diameter.

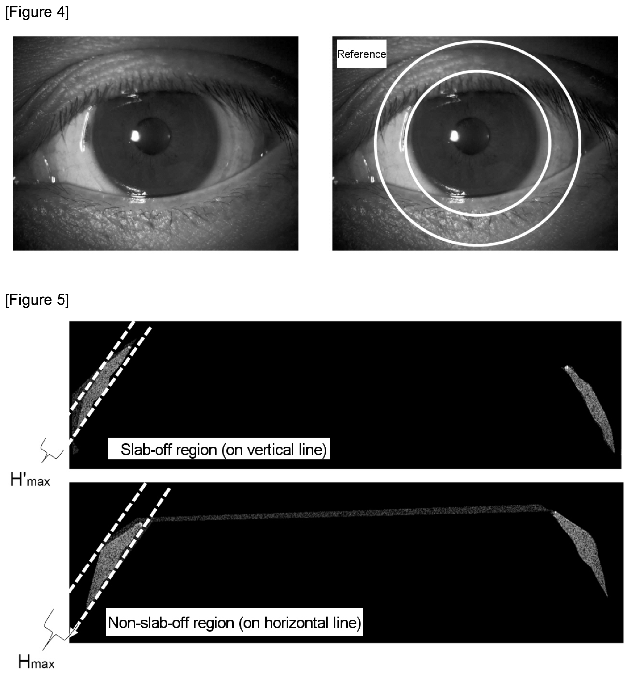

Figure 4 represents photographs showing the eye area taken from the front when wearing the annular device of Example 1 as described below. As shown in Figure 4, an annular device is worn on the scleral surface while exposing the cornea. As schematically shown by the two circular lines in the reference photograph of Figure 4, the upper and lower end sides of the annular device normally lie within the upper and lower eyelids, and frictional forces are generated between the upper and lower ends of the annular device and the eyelid conjunctiva during blinking. Due to such a structure, the annular device is prone to misalignment on the eyeball, making it difficult to stably hold the annular device in position.

Therefore, the annular device according to one embodiment of the present invention has a thin-layered slab-off region on the upper end side, or the upper and lower end sides of the annular device, thereby reducing frictional forces generated between the eyelid conjunctiva and the annular device during blinking to suppress misalignment of the annular device on the eyeball, resulting in excellent stability.

Figure 5 represents cross-sectional images obtained by cutting the annular device of Example 13 along a vertical line and a horizontal line, respectively, and photographed by optical coherence tomography (OCT). As shown in Figure 5, the maximum thickness H'max of the slab-off region is smaller than the maximum thickness Hmax of the non-slab-off region. With the above slab-off region, the annular device according to one embodiment of the present invention may exhibit excellent stability on the eyeball.

The basic configuration of the annular device may be large enough to cover the scleral surface without interfering with being worn. While the size of the annular device may be appropriately set, examples of the outer diameter of the annular device include preferably 16 mm to 22 mm, and more preferably 18 mm to 20 mm, in view of ease of insertion onto the eye, wearability, and coverage of the scleral surface. On the other hand, examples of the inner diameter of the annular device (diameter of the opening) include preferably 10 mm to 15 mm, in view of preventing the inner edge of the annular device from contacting the cornea.

The base curve (BC) of the annular device may be appropriately set, as long as the annular device can adhere to the scleral surface. Examples of the base curve include preferably 9.5 mm to 18.0 mm, and more preferably 10.0 mm to 16.0 mm, from the viewpoint of adhesion on the scleral surface. The base curve of a conventional contact lens is 8.0 mm to 9.0 mm.

The annular device may have at least one approximately encircling groove on the back surface side in contact with the scleral surface. When the annular device having the approximately encircling groove on the back surface side is worn, negative pressure is generated in the approximately encircling groove, thereby suppressing movement of the annular device on the scleral surface and improving stability of the annular device on the eyeball. The approximately encircling groove is formed to travel in a circumferential direction (this direction is also referred to as an encircling direction) in the annular device. The shape of the approximately encircling groove is not particularly limited. Thus, the approximately encircling groove may be a closed circular groove formed to travel entirely in the encircling direction; and a groove formed to travel partially or intermittently in the encircling direction. Alternatively, the approximately encircling groove may be formed in a wavy shape to travel in the encircling direction. The shape of the section of the approximately encircling groove may be, for example, semicircle or polygons such as triangle and quadrangle, preferably semicircle. While the number of approximately encircling grooves may be appropriately set, examples of the number include preferably one or more, and from the viewpoint that a larger number of grooves may raise a problem in terms of strength of the annular device, more preferably one to five, and even more preferably one to three.

The first form of annular device is described below with reference to Figures 1A and 1B, and the second form of annular device is described below with reference to Figures 2A and 2B. It is noted that a color gradation is shown in these figures, and that a region indicated by a relatively darker color corresponds to the slab-off region, and the maximum thickness gradually decreases as the color becomes darker. For example, in Figure 1A, the color becomes darker from a boundary point A1 toward a boundary point X1, indicating that the maximum thickness gradually decreases from the boundary point A1 toward the boundary point X1. In other words, in the slab-off region, the maximum thickness is not uniform from the boundary point A1 toward the boundary point X1, but has a gradient from large toward small.

### [First form of annular device]

Figures 1A and 1B represent plan views and cross-sectional views according to one embodiment of the first form of annular device (annual device 1). In Figure 1A, the body portion of the annular device 1 is comprised of a slab-off region 21, and a non-slab-off region 31 that is a region other than the slab-off region within the body portion. The slab-off region 21 extends in a clockwise direction from a line segment A1-A'1 toward a line segment B1-B'1. The line segment A1-A'1 connects an exterior tangent point A1 on the outer peripheral edge and an interior tangent point A'1 on the inner peripheral edge, the interior tangent point A'1 being positioned between the exterior tangent point A1 and a center point O1. The line segment B1-B'1 connects an exterior tangent point B1 on the outer peripheral edge and an interior tangent point B'1 on the inner peripheral edge, the interior tangent point B'1 being positioned between the exterior tangent point B1 and the center point O1. The annular device 1 has an asymmetric structure when a horizontal line HL1 is the axis of symmetry.

The slab-off region 21 is formed such that a maximum thickness is smallest at a line segment X1-X'1. The line segment X1-X'1 connects an exterior tangent point X1 on the outer peripheral edge in the upper end and an interior tangent point X'1 on the inner peripheral edge, the interior tangent point X'1 being positioned between the exterior tangent point X1 and the center point O1. The maximum thickness refers to a thickness of the thickest portion in the line segment connecting an exterior tangent point on the outer peripheral edge and an interior tangent point on the inner peripheral edge, the interior tangent point being positioned between the exterior tangent point and the center point.

The maximum thickness of the slab-off region 21 is configured to be smaller than a maximum thickness of the non-slab-off region 31. Specifically, the slab-off region 21 is configured such that the maximum thickness is smaller than a maximum thickness at a line segment Y1-Y'1. The line segment Y1-Y'1 connects an exterior tangent point Y1 on the outer peripheral edge in the lower end and an interior tangent point Y'1 on the inner peripheral edge, the interior tangent point Y'1 being positioned between the exterior tangent point Y1 and the center point O1.

The maximum thickness of the non-slab-off region 31 is substantially uniform. Even when the non-slab region 31 has some variation in maximum thickness, the maximum thickness at any portion of the non-slab-off region 31 is greater than the maximum thickness at any portion of the slab-off region 21. For example, the maximum thickness of the non-slab-off region 31 may be defined at the line segment Y1-Y'1 and may be slightly reduced from the line segment Y1-Y'1 toward the line segment A1-A'1 and/or the line segment B1-B'1, provided that the thickness at any portion of the non-slab-off region 31 is greater than the maximum thickness at any portion of the slab-off region 21.

As shown in Figure 1A, the annular device 1 has a gradient such that the maximum thickness is smallest at the line segment X1-X'1, which gradually decreases in a clockwise direction from the line segment A1-A'1 toward the line segment X1-X'1, and in a counterclockwise direction from the line segment B1-B'1 toward the line segment X1-X'1.

The maximum thicknesses of the slab-off region 21 and the non-slab-off region 31 may be appropriately set, provided that the maximum thickness of the slab-off region 21 is smaller than the maximum thickness at any portion of the non-slab-off region 31. Examples of the maximum thickness of the slab-off region 21 include preferably 50 µm to 500 µm, more preferably 80 µm to 400 µm, and even more preferably 100 µm to 200 µm, from the viewpoint of reducing frictional forces generated by rubbing between the back side of the upper eyelid and the surface of the annular device when worn. Examples of the maximum thickness of the non-slab-off region 31 include preferably 150 µm to 700 µm, more preferably 200 µm to 600 µm, and even more preferably 200 µm to 500 µm, from the viewpoint of compensating for reduced shape retention of the annular device due to the slab-off region configured.

In order to more effectively suppress displacement and rotational movement due to blinking when wearing the annular device, the ratio of the maximum thickness of the non-slab-off region 31 relative to the maximum thickness of the line segment X1-X'1 in the slab-off region 21 is preferably 100% to 500%, more preferably 120% to 450%, and even more preferably 150% to 400%.

The slab-off region 21 preferably has a line-symmetrical configuration with respect to a vertical line VL1 as an axis of symmetry. In this embodiment, the exterior tangent point A1 is line-symmetrical to the exterior tangent point B1 with respect to the vertical line VL1 as an axis of symmetry, while the interior tangent point A'1 is line-symmetrical to the interior tangent point B'1 with respect to the vertical line VL1 as an axis of symmetry. Hereinafter, an embodiment in which the slab-off region 21 has a line-symmetrical configuration with respect to the vertical line VL1 as an axis of symmetry will be described.

When a line segment X1-O1 connecting the exterior tangent point X1 and the center point O1 is taken as a starting line and a line segment B1-O1 connecting the exterior tangent point B1 and the center point O1 is taken as a moving radius, the angle measured in a clockwise direction from the starting line toward the moving radius is defined as a center angle P1. The center angle P1, which is a starting point of the slab-off region 21 in the body portion of the annular device 1, may be 40° to 170°. Examples of the center angle P1 include, from the viewpoint of reducing frictional forces generated between the back side of the upper eyelid and the surface of the annular device during blinking, preferably 60° to 160°, and more preferably 75° to 155°. Similarly, when the line segment X1-O1 is taken as the starting line and a line segment A1-O1 connecting the exterior tangent point A1 and the center point O1 is taken as a moving radius, an angle measured in a counterclockwise direction from the starting line toward the moving radius corresponding to a starting point of the slab-off region 21 in the body portion of the annular device 1 may be 40° to 170°, for example, preferably 60° to 160°, and more preferably 75° to 155°.

Figure 1B represents cross-sectional views of the body portion of the annular device 1 at respective center angles, for a specific embodiment in which the starting point of the slab-off region is located at a position corresponding to a center angle of 135°. As shown in Figure 1B, the maximum thickness gradually increases from the line segment X1-X'1 having a center angle of 0° (and 360°) toward the line segment B1-B'1 having a center angle of 135°, which corresponds to a starting point of the slab-off region 21. The region having a center angle of 135° to 225° corresponds to the non-slab-off region having a substantially uniform maximum thickness. Furthermore, the region having a center angle of 225° to 360° corresponds to the slab-off region in which the maximum thickness gradually decreases.

As long as the annular device 1 employs the configuration described above, other configurations of the annular device 1 may be appropriately set. On the other hand, the annular device 1 preferably has a predetermined relationship between surface areas of the slab-off region 21 and the non-slab-off region 31, from the viewpoint of reducing frictional forces generated between the eyelid conjunctiva and the annular device 1 during blinking when the annular device 1 is worn, thereby suppressing misalignment and rotational movement of the annular device 1 while improving handling by allowing the annular device 1 to retain a certain shape and to be easy to wear. Examples of the surface area of the slab-off region 21 include preferably 20% to 90%, more preferably 25% to 70%, and even more preferably 30% to 60% of the whole surface area of the body portion of the annular device 1 (total surface area of the slab-off region 21 and non-slab-off region 31).

The annular device 1 is preferably configured such that the non-slab-off region 31 and/or the slab-off region 21 have a maximum thickness in an intermediate portion between inner and outer edge portions.

For example, the non-slab-off region 31 preferably has a continuous configuration comprised of an inner edge portion 311, an intermediate portion 312, and an outer edge portion 313, such that the intermediate portion includes a portion having a maximum thickness, and more preferably is configured such that the thickness gradually decreases from the portion having the maximum thickness in the intermediate portion toward the outer peripheral edge.

Similarly, for example, the slab-off region 21 preferably has a continuous configuration comprised of an inner edge portion 211, an intermediate portion 212, and an outer edge portion 213, such that the intermediate portion includes a portion having a maximum thickness, and more preferably is configured such that the thickness gradually decreases from the portion having the maximum thickness in the intermediate portion toward the outer peripheral edge. However, depending on the maximum thickness in the intermediate portion 212, the slab-off region 21 may be too thin at the inner edge portion 211 and the outer edge portion 213, which may result in poor shape retention and handling of the annular device. Therefore, the inner edge portion 211, the intermediate portion 212, and the outer edge portion 213 may have a uniform thickness.

The widths of portions corresponding to inner and outer edge portions of the annular device 1 may be set as appropriate. Examples of the width of the inner edge portion 211 or 311 include preferably 0.3 mm to 0.5 mm in a radial direction from the interior tangent point on the inner peripheral edge of the opening side of the annular device 1 toward the outer peripheral edge side. Examples of the width of the outer edge portion 213 or 313 include preferably 0.3 mm to 0.5 mm in a radial direction from the exterior tangent point on the outer peripheral edge of the annular device 1 toward the inner peripheral edge side. The intermediate portions 212 and 312 are portions of the annular device 1 excluding the inner edge portion and the outer edge portion.

### [Second form of annular device]

Figures 2A and 2B represent plan views and cross-sectional views according to one embodiment of the second form of annular device (annual device 2). In Figure 2A, the body portion of the annular device 2 is comprised of a slab-off region 22 and a slab-off region 23, and a non-slab-off region 32 and a non-slab-off region 33, which are regions other than the slab-off regions 22 and 23 within the body portion. The slab-off region 22 extends in a clockwise direction from a line segment A2-A'2 toward a line segment B2-B'2. The line segment A2-A'2 connects an exterior tangent point A2 on the outer peripheral edge and an interior tangent point A'2 on the inner peripheral edge, the interior tangent point A'2 being positioned between the exterior tangent point A2 and a center point O2. The line segment B2-B'2 connects an exterior tangent point B2 on the outer peripheral edge and an interior tangent point B'2 on the inner peripheral edge, the interior tangent point B'2 being positioned between the exterior tangent point B2 and the center point O2. The slab-off region 23 is line-symmetrical to the slab-off region 22 with respect to a horizontal line HL2 as an axis of symmetry.

The slab-off region 22 is configured such that a maximum thickness is smallest at a line segment X2-X'2 connecting an exterior tangent point X2 and an interior tangent point X'2.

The maximum thickness of the slab-off region 22 is configured to be smaller than the maximum thickness of the non-slab-off region 32. Specifically, the slab-off region 22 is configured such that the maximum thickness is smaller than maximum thicknesses at a line segment Y2-Y'2 and a line segment Y3-Y'3. The line segment Y2-Y'2 connects an exterior tangent point Y2 and an interior tangent point Y'2, while the line segment Y3-Y'3 connects an interior tangent point Y3 and an interior tangent point Y'3.

The maximum thickness of the non-slab-off region 32 is substantially uniform. Even when the non-slab region 32 has some variation in maximum thickness, the maximum thickness at any portion of the non-slab-off region 32 is greater than the maximum thickness at any portion of the slab-off region 22. For example, the maximum thickness of the non-slab-off region 32 may be defined at the line segment Y2-Y'2 and may be slightly reduced from the line segment Y2-Y'2 toward a line segment A2-A'2 and/or a line segment A3-A'3, provided that the thickness at any portion of the non-slab-off region 32 is greater than the maximum thickness at any portion of the slab-off region 22.

As shown in Figure 2A, the annular device 2 has a gradient such that the maximum thickness is smallest at the line segment X2-X'2, in a clockwise direction from the line segment A2-A'2 toward the line segment X2-X'2, and in a counterclockwise direction from the line segment B2-B'2 toward the line segment X2-X'2.

The maximum thicknesses of the slab-off region 22 and the non-slab-off region 32 may be appropriately set, provided that the maximum thickness of the slab-off region 22 is smaller than the maximum thickness at any portion of the non-slab-off region 32. Examples of the maximum thickness of the slab-off region 22 include preferably 50 µm to 500 µm, more preferably 80 µm to 400 µm, and even more preferably 100 µm to 400 µm, from the viewpoint of reducing frictional forces generated by rubbing between the back side of the upper eyelid and the surface of the annular device when worn. Examples of the maximum thickness of the non-slab-off region 32 include preferably 150 µm to 700 µm, more preferably 200 µm to 600 µm, even more preferably 300 µm to 600 µm, and still even more preferably 350 µm to 550 µm, from the viewpoint of compensating for reduced shape retention of the annular device due to the slab-off region configured.

In order to more effectively suppress displacement and rotational movement due to blinking when wearing the annular device, the ratio of the maximum thickness of the non-slab-off region 32 relative to the maximum thickness of the line segment X2-X'2 in the slab-off region 22 is preferably 100% to 500%, more preferably 120% to 450%, and even more preferably 120% to 300%.

The slab-off region 22 preferably has a line-symmetrical configuration with respect to a vertical line VL2 as an axis of symmetry. In this embodiment, the exterior tangent point A2 is line-symmetrical to the exterior tangent point B2 with respect to the vertical line VL2 as an axis of symmetry, while the interior tangent point A'2 is line-symmetrical to the interior tangent point B'2 with respect to the vertical line VL2 as an axis of symmetry. Hereinafter, an embodiment in which the slab-off region 22 has a line-symmetrical configuration with respect to the vertical line VL2 as an axis of symmetry will be described.

When a line segment X2-O2 connecting the exterior tangent point X2 and the center point O2 is taken as a starting line and a line segment B2-O2 connecting the exterior tangent point B2 and the center point O2 is taken as a moving radius, the angle measured in a clockwise direction from the starting line toward the moving radius is defined as a center angle P2. The center angle P2, which is a starting point of the slab-off region 22 in the body portion of the annular device 2, may be 40° to 85°. Examples of the center angle P2 include, from the viewpoint of reducing frictional forces generated between the back side of the upper eyelid and the surface of the annular device during blinking, preferably 50° to 85°, and more preferably 60° to 80°. Similarly, when the line segment X2-O2 is taken as the starting line and a line segment A2-O2 connecting the exterior tangent point A2 and the center point O2 is taken as a moving radius, an angle measured in a counterclockwise direction from the starting line toward the moving radius corresponding to a starting point of the slab-off region 22 in the body portion of the annular device 2 may be 40° to 85°, for example, preferably 50° to 85°, and more preferably 60° to 80°.

The annular device 2 has the slab-off region 23 that is line-symmetrical to the slab-off region 22 with respect to the horizontal line HL2 as an axis of symmetry. While the slab-off region 23 preferably has exactly the same configuration as the slab-off region 22, the slab-off region 23 may have a similar configuration to the slab-off region 22 in terms of maximum thickness, center angle, and the like. The annular device 2 has the non-slab-off region 33 that is line-symmetrical to the non-slab-off region 32 with respect to the vertical line VL2 as an axis of symmetry. While the non-slab-off region 33 preferably has exactly the same configuration as the non-slab-off region 32, the non-slab-off region 33 may have a similar configuration to the non-slab-off region 32 in terms of maximum thickness, center angle, and the like.

As long as the annular device 2 employs the configuration described above, other configurations of the annular device 2 may be appropriately set. On the other hand, the annular device 2 preferably has a predetermined relationship between surface areas of the slab-off regions 22 and 23, and surface areas of the non-slab-off regions 32 and 33, from the viewpoint of reducing frictional forces generated between the eyelid conjunctiva and the annular device 2 during blinking, thereby suppressing misalignment and rotational movement of the annular device 2 while improving handling by allowing the annular device 2 to retain a certain shape and to be easy to wear. Examples of the total surface area of the slab-off regions 22 and 23 include preferably 20% to 90%, more preferably 25% to 85%, even more preferably 50% to 80%, and still even more preferably 60% to 80% of the whole surface area of the body portion of the annular device 2 (total surface area of the slab-off regions 22 and 23 and the non-slab-off regions 32 and 33).

The annular device 2 is preferably configured such that the non-slab-off region 32 and/or the non-slab-off region 33, the slab-off region 22 and/or the slab-off region 23 have a maximum thickness in an intermediate portion between inner and outer edge portions.

For example, the non-slab-off region 32 preferably has a continuous configuration comprised of an inner edge portion 321 (or 331), an intermediate portion 322 (or 332), and an outer edge portion 323 (or 333), such that the intermediate portion includes a portion having a maximum thickness, and more preferably is configured such that the thickness gradually decreases from the portion having the maximum thickness in the intermediate portion toward the outer peripheral edge.

Similarly, for example, the slab-off region 22 preferably has a continuous configuration comprised of an inner edge portion 221, an intermediate portion 222, and an outer edge portion 223, such that the intermediate portion includes a portion having a maximum thickness, and more preferably is configured such that the thickness gradually decreases from the portion having the maximum thickness in the intermediate portion toward the outer peripheral edge. However, depending on the maximum thickness in the intermediate portion 222, the slab-off region 22 may be too thin at the inner edge portion 221 and the outer edge portion 223, which may result in poor shape retention and handling of the annular device. Therefore, the inner edge portion 221, the intermediate portion 222, and the outer edge portion 223 may have a uniform thickness. The same may apply to the slab-off region 23.

The widths of portions corresponding to inner and outer edge portions of the annular device 2 may be set as appropriate. Examples of the width of the inner edge portion 221, 231, 321 or 331 include preferably 0.3 mm to 0.5 mm from the interior tangent point on the inner peripheral edge of the opening side of the annular device 2 toward the outer peripheral edge side. Examples of the width of the outer edge portion 223, 233, 323 or 333 include preferably 0.3 mm to 0.5 mm from the exterior tangent point on the outer peripheral edge of the annular device 2 toward the inner peripheral edge side. The intermediate portions 222, 232, 322 and 332 are portions of the annular device 2 excluding the inner edge portion and the outer edge portion.

Figure 2B represents one embodiment of the second form of annular device having two approximately encircling grooves on the back surface.

### [Material of annular device]

The annular device according to one embodiment of the present invention is comprised of a hydrogel. The hydrogel is based on a polymerized compound (polymer) of monomer components including a hydrophilic monomer. The hydrogel is swollen by an aqueous solution such as water or a water-containing solution and retains the aqueous solution in the polymer matrix. The polymer (polymerized compound) is produced by polymerization of monomers, and the constituent unit derived from each monomer in the polymer is also referred to as a portion or residue.

Specific examples of the hydrogel include hydrogel produced with hydrophilic monomers, hydrogel produced with hydrophilic monomers and monomers copolymerizable with hydrophilic monomer such as hydrophobic monomers and cross-linking monomers.

The hydrophilic monomer may improve the water content of the resulting hydrogel. The hydrophobic monomer may adjust a water content and a swelling rate of the resulting hydrogel, and may also slightly adjust an amount of drug contained when the annular device is a drug-containing annular device.

The cross-linking monomer may control the density of polymer chains in the resulting hydrogel depending on its content, and such control of cross-linking density may inhibit drug diffusion and delay release of a contained drug, thereby controlling a drug release rate. The cross-linking monomer may control a drug release rate and may also impart properties such as mechanical strength, shape stability, and solvent resistance to the resulting hydrogel.

The hydrophilic monomer may be a monomer having in the molecule at least one hydrophilic group. Examples of the hydrophilic monomer include 2-hydroxyethyl (meth)acrylate, 2-hydroxymethyl (meth)acrylate, hydroxypropyl (meth)acrylate, 2-hydroxybutyl (meth)acrylate, glycerol (meth)acrylate, acrylamide, N,N-dimethyl (meth)acrylamide, N,N-diethyl (meth)acrylamide, N-vinylpyrrolidone, diacetone acrylamide, N-vinylacetamide, (meth)acrylic acid, (meth)acryloxyethyl succinic acid, itaconic acid, methacrylamidopropyltrimonium chloride, and 2,3-dihydroxypropyl (meth)acrylate. The hydrophilic monomer may be used either individually or in combination of two or more of those listed above. The hydrophilic monomer is preferably 2-hydroxyethyl (meth)acrylate, since the monomer can be copolymerized with various monomers and is highly versatile. In addition, (meth)acrylate is a generic term for both acrylate and methacrylate.

The content of the hydrophilic monomer may be appropriately set. Examples of the content include preferably 50% by weight or more of the total monomer component from the viewpoint of imparting sufficient hydrophilicity to the annular device. A content of the hydrophilic monomer of less than 50% by weight is not preferred, since such a content may fail to impart sufficient hydrophilicity to the annular device, thereby decreasing flexibility of the annular device.

The hydrophobic monomer may be a monomer having no hydrophilic group, or a monomer that is evaluated to be hydrophobic as a whole even if the monomer has a hydrophilic group (i.e., a monomer different from the hydrophilic monomer). Examples of the hydrophobic monomer include siloxanyl (meth)acrylate, trifluoroethyl (meth)acrylate, methacrylamide, cyclohexyl (meth)acrylate, n-butyl (meth)acrylate, lauryl (meth)acrylate, and tetrahydrofurfuryl acrylate. The hydrophobic monomer may be used either individually or in combination of two or more of those listed above.

The content of the hydrophobic monomer may be appropriately set. The hydrophobic monomer may change the water content of the annular device depending on its content. A higher content of the hydrophobic monomer may cause a significant decrease in the water content of the annular device, thereby decreasing flexibility of the annular device. Therefore, the content of the hydrophobic monomer is preferably less than 30% by weight of the total monomer component.

The cross-linking monomer may be a monomer having two or more polymerizable groups. Examples of the cross-linking monomer include (meth)acrylate-based cross-linking compounds such as ethylene glycol di(meth)acrylate, diethylene glycol di(meth)acrylate, triethylene glycol di(meth)acrylate, propylene glycol di(meth)acrylate, dipropylene glycol di(meth)acrylate, trimethylolpropane tri(meth)acrylate, and pentaerythritol tri(meth)acrylate; vinyl-based cross-linking compounds such as allyl methacrylate, diarylmaleate, diarylfumarate, diaryl succinate, diaryl phthalate, triallyl cyanurate, triallyl isocyanurate, diethylene glycol bisaryl carbonate, triallyl phosphate, triallyltrimellitate, diaryl ether, N,N-diaryl melamine, and divinylbenzene. The cross-linking monomer may be used either individually or in combination of two or more of those listed above in order to impart desired properties to the annular device.

The content of the cross-linking monomer may be appropriately set. Examples of the content include preferably 0.1% by weight to 10% by weight of the total monomer content from the viewpoint of modulating the shape of the annular device. If the content of the cross-linking monomer is less than 0.1% by weight, the cross-linked network structure of the annular device may be insufficient. If the content exceeds 10% by weight, the cross-linked network structure may be excessive, resulting in reduced flexibility and increased fragility of the annular device.

Various additives may be added to the annular device before or after the copolymerization reaction of the monomers. The additives may be appropriately selected. Examples of the additives include poly-N-vinyl-2-pyrrolidone (PVP), polyethylene glycol (PEG), polyvinyl alcohol (PVA), poly(N,N-dimethylacrylamide) (PDMAA), polyacrylic acid (PAA), polyhydroxyethyl methacrylate (PHEMA), carboxybetaine-type polymer, phosphobetaine-type polymer, sulfobetaine-type polymer, polyglycolic acid (PGA), and polylactic acid (PLA). The additives may be used either individually or in combination of two or more of those listed above in order to impart desired properties to the annular device. The additives are preferably PVP, PEG, betaine-type polymer, PHEMA, PGA, and PLA. The amount of the additives added may be appropriately set in view of imparting desired properties to the annular device.

A polymerization initiator may be employed as one of the additives to initiate polymerization of the monomers. Examples of the polymerization initiator include common radical polymerization initiators, e.g., peroxides such as lauroyl peroxide, cumene hydroperoxide, and benzoyl peroxide; azobisvaleronitrile, and azobisisobutyronitrile. The polymerization initiator may be used either individually or in combination of two or more of those listed above. The amount of the polymerization initiator added may be an amount that promotes polymerization of the monomers. Examples of the amount include preferably about 10 ppm to about 3,500 ppm relative to the total monomer content.

A UV absorber may be employed as one of the additives to impart a UV absorbing effect to the annular device. Examples of the UV absorber include 2-hydroxy-4-(meth)acryloyloxybenzophenone, 2-hydroxy-4-(meth)acryloyloxy-5-t-butylbenzophenone, 2-(2'-hydroxy-5'-(meth)acryloyloxyethylphenyl)-2H-benzotriazole, 2-(2'-hydroxy-5'-(meth)acryloyloxyethylphenyl)-5-chloro-2H-benzotriazole, and 2-hydroxy-4-methacryloyloxymethylbenzoic acid phenyl ester. The UV absorber may be used either individually or in combination of two or more of those listed above. The UV absorber may be added in an amount sufficient to impart a desired UV absorption effect to the annular device.

### [Method of producing annular device]

The annular device according to one embodiment of the present invention may be produced by applying conventional production methods used for contact lenses, such as a cast molding process, and a lathe cut process.

For example, the cast molding process is a process including using a mold that is predesigned to have a desired shape (annular form) after polymerization, and carrying out polymerization of monomer components in the mold to obtain an annular device. The lathe cut process is a process including obtaining a block-shaped polymer, and cutting and polishing the resulting block into the shape of an annular device to obtain the annular device. The cast molding process and the lathe cut process are described in detail below, but are not limited to them with respect to the method of producing an annular device.

### Cast molding process

The polymerization initiator is added to a mixture of monomers including the hydrophilic monomer, or the hydrophilic monomer and monomers copolymerizable with the hydrophilic monomer, such as the hydrophobic monomer and the cross-linking monomer. The mixture is stirred to dissolve the components, thereby obtaining a monomer mixture solution.

The monomer mixture solution is placed in a mold made of metal, glass, plastic, or other material, and then the mold containing the monomer mixture solution is sealed and heated in a thermostatic bath or other device, stepwise or continuously, at a temperature of 25 °C to 130 °C for 5 hours to 120 hours to carry out polymerization reaction. In the polymerization reaction, photopolymerization using ultraviolet rays, electron beams, or gamma rays may also be employed. Solution polymerization may also be applied by adding water or an organic solvent to the monomer mixture solution.

After the polymerization reaction, the mold is cooled to room temperature, and the resulting polymer is removed from the mold and, if necessary, subjected to cutting and polishing. The polymer is then hydrated and swollen with an aqueous liquid to form a hydrous gel (hydrogel) to obtain an annular device. Examples of the aqueous liquid (swelling liquid) used for hydration and swelling include water, a saline solution, and an isotonic buffer solution. The swelling liquid may also be a mixture with the aqueous liquid and a water-soluble organic solvent. The hydration swelling treatment may be preferably performed by immersing the polymer in the swelling solution warmed to 40 °C to 100 °C for a certain period of time to immediately put the polymer in a hydration swollen state. In the hydration swelling treatment, it is possible to remove unpolymerized monomers contained in the polymer.

### Lathe cut process

Using a mold designed to form a block-shaped polymer after polymerization, a block-shaped polymer is obtained in a manner similar to the cast molding process. The resulting block-shaped polymer is then cut to produce a scleral lens configured to cover both the sclera and cornea. The resulting scleral lens is processed to have an opening configured to expose the cornea, and outer and inner peripheral edges thereof are polished to obtain an annular device. In this case, the polymer is first processed to form the opening and is then cut into a ring-shaped device.

### [Usage of annular device]

The annular device according to one embodiment of the present invention is worn such that the annular device can cover the sclera, i.e., on the surface of the sclera, more specifically, on the surface of the bulbar conjunctiva covering the sclera. For wearing, a dedicated auxiliary tool may be used; alternatively, the annular device may be held by pinching with fingers, placed on the periphery of the bulbar conjunctiva, and then fitted by gently adapting with the fingers so as to cover the bulbar conjunctiva.

The annular device according to one embodiment of the present invention may be used as an annular device for sustained drug release. In this case, the dosage of the drug administered to the ocular tissue by the annular device may be appropriately set depending on the type of the drug. For example, the dosage may be usually from about 1 µg to about 100 mg per administration. The number of administrations of the drug, i.e., the frequency of wearing the annular device according to one embodiment of the present invention, may be appropriately selected according to symptoms, ages, or other factors. Examples of the frequency include once to multiple times per day (e.g., once to six times). The annular device may be worn in the eye once every few days to once every few months.

The present invention will now be described in further detail with reference to Examples, which are not intended to limit the present invention. The present invention may take various embodiments to the extent that the objectives of the present invention are achieved.

### Examples

### [Preparation method of annular device]

A monomer mixture solution was obtained by mixing 99 g of 2-hydroxyethyl methacrylate as the hydrophilic monomer, 1 g of ethylene glycol dimethacrylate as the cross-linking monomer, and 0.15 g of 2,2'-azobisisobutyronitrile as the polymerization initiator. The monomer mixture solution was placed into molds designed to produce the annular devices of Examples 1 to 13 and Comparative Example 1 having the sizes and structures shown in Table 1. The mold containing the monomer mixture solution was subjected to heat polymerization treatment (under a nitrogen atmosphere, with the temperature being increased from room temperature to 100 °C over 40 hours). After the heat polymerization treatment, the polymer removed from the mold was subjected to swelling treatment by heating the polymer in a saline solution at 60 °C for 30 minutes, and was then subjected to high-pressure steam sterilization treatment to obtain an annular device. The thickness of the slab-off region in Table 1 refers to a maximum thickness at the line segment X1-X'1 in Figure 1A or at the line segment X2-X'2 in Figure 2A. The thickness of the non-slab-off region in Table 1 refers to a maximum thickness at the line segment Y1-Y'1 in Figure 1A or at the line segment Y2-Y'2 in Figure 2A. Examples 1 to 13 have a line symmetrical structure with respect to the vertical line VL1 or VL2 as the axis of symmetry. Examples 6 to 13 have a line symmetrical structure with respect to the horizontal line HL2 as the axis of symmetry.

The thickness of the annular device was measured using the measurement mode of a digital microscope ("VHX-6000"; Keyence) on a section of the annular device cut in a radial direction.

### [Sensory evaluation method]

The annular devices of Examples 1 to 13 and Comparative Example 1 were worn by subjects, and the wearing sensation experienced by each subject was evaluated according to the following criteria. Similarly, the inside of the eye of the subject after wearing the annular device was observed using a slit-lamp microscope, and the stability of the annular device on the eyeball was evaluated according to the following criteria.

### Wearing sensation

++: No foreign body sensation and good wearing sensation
+: Slight foreign body sensation, with no interference with wearing
±: Moderately strong foreign body sensation, but the annular device is wearable
-: Foreign body sensation, making the annular device unwearable

### Stability on eyeball

+: Stably holding of the annular device in the center of the eye during blinking
-: Slipping of the annular device out of the eye or holding of the annular device completely over the pupil during blinking

In addition, the ease of handling (ease of wearing) of the annular device when being worn was evaluated according to the following criteria.

### Ease of handling

++: Easy to be worn
+: Taking some time for wearing
±: Taking much time for wearing
-: Unwearable

### <Evaluation results>

Table 1 shows the sizes and structures of the annular devices, and the results of the sensory evaluation. The annular devices of Examples 1 to 13 included at least a slab-off region, thereby improving both wearing sensation and stability on the eyeball, as well as ease of handling. On the other hand, it was found that the annular device of Comparative Example 1 was ring-shaped with no slab-off region, resulting in poorer scores in all sensory evaluation items, particularly in stability on the eyeball.

In Examples 1 to 5, the annular device having a thickness of the lower non-slab-off region of 0.35 mm or greater was less deformation during handling, resulting in improved ease of handling. On the other hand, the annular device having a maximum thickness ratio between the slab-off and non-slab-off regions of 300% or less had less effect on the eyelid during blinking, resulting in improved wearing sensation.

In Examples 6 to 13, the annular devices having a non-slab-off region thickness of 0.50 mm and a maximum thickness ratio between the slab-off and non-slab-off regions of 150% or less exhibited improved overall holding sensation and ease of handling, and surprisingly improved wearing sensation.

Conventional contact lenses had a thickness of 0.15 mm. The annular devices described in Patent Documents 1 and 2 had a maximum thickness of 0.25 mm to 0.38 mm. It was found that the annular device having the slab-off region and further having a maximum thickness of the non-slab-off region of 0.40 mm or more exhibited improved ease of handling, wearing sensation, and stability on the eyeball.

**[Table 1]**

| | Outer diameter (mm) | Inner diameter (mm) | BC (mm) | Encircling groove (number) | Slab-off region | | | | Non-slab-off region | | | | Wearing sensation | Stability on eyeball | Ease of handling |
|---|---|---|---|---|---|---|---|---|---|---|---|---|---|---|---|
| | | | | | Position | Configuration | | | Position | Configuration | | | | | |
| | | | | | | Thickness (mm) | Center angle (° ) | Surface area (%) | | Thickness (mm) | Surface area (%) | Thickness ratio (%) | | | |
| Example 1 | 20 | 14 | 15 | 2 | Upper only | 0.12 | 90 | 35 | Lower | 0.45 | 65 | 375 | ± | + | ++ |
| Example 2 | 20 | 14 | 15 | 2 | Upper only | 0.12 | 90 | 35 | Lower | 0.35 | 65 | 292 | + | + | ++ |
| Example 3 | 20 | 14 | 15 | 2 | Upper only | 0.12 | 90 | 35 | Lower | 0.25 | 65 | 208 | + | + | + |
| Example 4 | 20 | 14 | 15 | 2 | Upper only | 0.12 | 120 | 40 | Lower | 0.35 | 60 | 292 | + | + | ++ |
| Example 5 | 20 | 14 | 15 | 2 | Upper only | 0.12 | 150 | 58 | Lower | 0.35 | 42 | 292 | + | + | ++ |
| Example 6 | 20 | 14 | 15 | 2 | Upper and lower | 0.15 | 75 | 68 | Right and left | 0.35 | 32 | 233 | + | + | + |
| Example 7 | 20 | 14 | 14 | 2 | Upper and lower | 0.15 | 75 | 68 | Right and left | 0.35 | 32 | 233 | + | + | + |
| Example 8 | 18 | 14 | 11 | 2 | Upper and lower | 0.23 | 75 | 68 | Right and left | 0.40 | 32 | 174 | + | + | + |
| Example 9 | 19 | 13 | 12 | 0 | Upper and lower | 0.17 | 75 | 68 | Right and left | 0.40 | 32 | 174 | + | + | + |
| Example 10 | 18.3 | 14 | 13 | 2 | Upper and lower | 0.20 | 75 | 65 | Right and left | 0.40 | 35 | 200 | + | + | + |
| Example 11 | 18.3 | 14 | 13 | 2 | Upper and lower | 0.30 | 75 | 65 | Right and left | 0.50 | 35 | 167 | + | + | + |
| Example 12 | 18.3 | 14 | 13 | 2 | Upper and lower | 0.25 | 75 | 65 | Right and left | 0.50 | 35 | 200 | ++ | + | + |
| Example 13 | 18.3 | 14 | 13 | 2 | Upper and lower | 0.35 | 75 | 65 | Right and left | 0.50 | 35 | 143 | ++ | + | ++ |
| Comparative Example 1 | 18 | 14 | 13 | 2 | - | - | - | - | - | - | - | - | ± | - | ± |

| | | | | | | | | | | | | | | | |
|---|---|---|---|---|---|---|---|---|---|---|---|---|---|---|---|
| 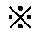 Central angle corresponds to one angle in upper or lower position. | | | | | | | | | | | | | | | |

### Description of symbols

1: First form of annular device
2: Second form of annular device
21, 22, 23: Slab-off region
211, 221, 231: Inner edge portion of slab-off region
212, 222, 232: Intermediate portion of slab-off region
213, 223, 233: Outer edge portion of slab-off region
31, 32, 33: Non-slab-off region
311, 321, 331: Inner edge portion of non-slab-off region
312, 322, 332: Intermediate portion of non-slab-off region
313, 323, 333: Outer edge portion of non-slab-off region

### [Industrial applicability]

According to the present invention, it is possible to provide an annular device that can reduce frictional forces generated between the annular device and the eyelid conjunctiva during blinking when worn, that can suppress the effect of such frictional forces, and that can exhibit excellent stability on the eyeball sufficient to suppress misalignment when worn, resulting in reducing foreign body sensation when worn and occurrence of ocular disorders. Furthermore, according to the present invention, by using the annular device as a drug-releasing annular device, it is possible to deliver the drug to a desired site on the eye. As a result, the present invention can contribute to health and welfare of living individuals.

### Cross-reference of related applications

The present application claims the benefit of priority to Japanese Patent Application No. 2023-180706, filed on October 20, 2023, the disclosure of which is incorporated herein by reference in its entirety. In addition, the disclosure of all the documents described herein including Patent Documents 1 to 3 is incorporated herein by reference in its entirety.

## Claims

1. An annular device configured to be worn over a surface of a sclera, comprising:
an opening configured to expose a cornea; and
a body portion having a slab-off region and a non-slab-off region,
wherein the slab-off region is formed such that a maximum thickness thereof gradually decreases from a lower end side toward an upper end side.

2. The annular device according to claim 1, wherein the slab-off region is line-symmetrical with respect to a vertical line as an axis of symmetry.

3. The annular device according to claim 2, wherein the slab-off region is formed such that a maximum thickness thereof gradually decreases from a lower end side toward an upper end side, starting from a position where a center angle is in a range of 40° to 170°.

4. An annular device configured to be worn over a surface of a sclera, comprising:
an opening configured to expose a cornea; and
a body portion having a slab-off region and a non-slab-off region,
wherein the slab-off region is formed such that a maximum thickness thereof gradually decreases from a right end side toward an upper end side and a lower end side, and from a left end side toward an upper end side and a lower end side.

5. The annular device according to claim 4, wherein the slab-off region is line-symmetrical with respect to a vertical line as an axis of symmetry.

6. The annular device according to claim 5, wherein the slab-off region is formed such that a maximum thickness thereof gradually decreases from a right end side toward an upper end side, starting from a position where a center angle is in a range of 40° to 85°, and from a right end side toward a lower end side, starting from a position where a center angle is in a range of 95° to 140°.

7. The annular device according to any one of claims 1 to 6, wherein the slab-off region has a surface area of 25% to 70% relative to a total surface area of the body portion.

8. The annular device according to any one of claims 1 to 6, wherein a maximum thickness of the non-slab-off region of the body portion is 120% to 450% relative to a minimum maximum thickness of the slab-off region.

9. The annular device according to any one of claims 1 to 6, wherein the body portion has one or more approximately encircling grooves on a back surface thereof.
